# EUROPEAN PATENT APPLICATION

(11) **EP 2 119 434 A1**
(43) Date of publication of application: **18.11.2009**
(21) Application number: 08156116.9
(22) Date of filing: 13.05.2008
(51) Int. Cl.: A61K 31/00, A61K 31/352, A61P 35/00

(54) **Use of heterosidic flavonoid derivatives for therapy of stem cell cancers**

(71) Applicant: INSTITUT NATIONAL DE LA SANTE ET DE LA RECHERCHE MEDICALE (INSERM), 75654 Paris Cédex 13 (FR)
(72) Inventor: The designation of the inventor has not yet been filed
(74) Representative: Le Coupanec, Pascale A.M.P.

(57) **Abstract**

The present invention relates to therapy of stem cell cancer and more specifically against acute myeloid leukaemia. The present invention more precisely deals with the use of heterosidic flavonoid derivatives and in particular rutin, or derivatives thereof for the treatment of stem cell cancer specifically acute myeloid leukaemia, for preventing tumor relapse in a patient and/or for preventing solid tumor metastasis in a patient.

## Description

### FIELD OF THE INVENTION:

The present invention relates to the therapy of cancers and more specifically of acute myeloid leukaemia. The present invention more precisely deals with the use of some flavonoid derivatives for their selective cytotoxicity towards cancer stem cells.

### BACKGROUND OF THE INVENTION :

Recently, rare populations of tumor cells designated as "tumor initiating cells" or "cancer stem cells" have been identified with the ability to drive tumor formation and supporting the cancer stem cell hypothesis. This hypothesis postulates that tumors arise from stem cells that drive tumorigenesis, giving rise to a large population of progenitors that make up the bulk of the tumor but would lack tumorigenic potential.

Properties of cancer stem cells mean that they are able to give rise to the original cancer by regenerating the tumor cells. They have been shown to be tumorigenic and resistant to radiotherapy in vitro. They are supposed to be resistant to treatments and therefore responsible of relapse after apparent tumor response in vivo. In cases in which tumor is eradicated by chemotherapy but is followed by a relapse, a possible reason is that the cancer stem cells are drug-resistant and have not been killed. Accordingly, therapeutic approaches that specifically target cancer stem cells should prevent or reduce the risk of relapse and metastasis.

Cancer stem cells have been identified first in acute and chronic leukemia, then in solid tumors such as breast cancers, brain tumors, prostate cancer and more recently in colon and head and neck cancers. They have been isolated using stem cell markers, some of which have been shown to be shared by different tissues, like the adhesion molecule CD44, detected in breast, prostate and head and neck cancer stem cells, and which is also expressed by normal healthy mammary tissue stem cells (Balic M, et al., Clin Cancer Res, 2006 12:5615-21).

Regarding acute myeloid leukemia (AML), it is characterized by the aberrant accumulation of immature myeloid hematopoietic cells. The leukemia-initiating cells are hematopoietic stem cells (HSCs) which have self-renewal and differentiation functions dysregulated or committed progenitors transformed to leukemic stem cells (LSCs). In all cases, the leukemic clone requires a proliferative and a survival advantage to overcome normal hematopoiesis.

Current chemotherapy regimens for AML use drugs such as nucleoside analogs and anthracyclines (eg, daunorubicin) that interfere with DNA replication and induce apoptosis primarily in replicating cells. However, since LSCs are mostly quiescent like HSCs, relapse is common after initial remission in response to chemotherapy.

The hematopoietic niche plays a key role in maintaining quiescence of HSCs. Adhesive interactions between HSCs and their microenvironment (osteoblasts, matrices, mesenchymal stem cells) are required to maintain stem cell capacities. Upon leukemic transformation, bone marrow presents enrichment in fibronectin and in hematopoietic cells overexpressing its receptor, the α5β1 integrin. The inventors and others have already demonstrated that adhesive interactions, notably through integrin engagement on fibronectin, were responsible for cell adhesion-mediated drug resistance (CAM-DR) of leukemic cells (De Toni F, et al.. Oncogene. 2006 May 25;25(22):3113-22). Accordingly, it is essential to determine specific cell survival mechanisms able to distinguish LSCs from HSCs to propose LSC-specific targeted therapy.

Few studies have demonstrated that LSCs do involve unique signaling pathways for their cell survival. Nuclear factor κB (NF-κB) and mammalian target of rapamycin (mTOR) were found constitutively activated in LSCs. Hence both the NF-κB inhibitor parthenolide from *Tanacetum parthenium* and the mTOR inhibitor rapamycin from *Streptomyces hygroscopicus* have been proposed as LSC-targeted therapies. The phosphoinositide-3 kinase/Akt pathway regulates NF-κB and mTOR activities, and mediates CAM-DR of AML. Another key signaling molecule controlling cell survival is the glycogene synthase kinase 3β (GSK3β), a serine threonine kinase important for the cell response to different stress from the microenvironment (serum, oxygen or glucose deprivation, oxidative or inflammatory stress). The pro-survival activity of GSK3β is tightly linked to the death receptors response (eg, TNF, TRAIL). Interestingly, GSK3β activation is linked to a cell quiescence status and regulates NF-κB through an original pathway independent of Iκ-B. This pathway allows the transcription of specific genes such as IL-6, a cytokine involved in the survival of malignant hematopoietic cells (Hoeflich KP, et al.. Nature. 2000 Jul 6;406(6791 ):86-90, and Steinbrecher KA, et al.. Mol Cell Biol. 2005 Oct;25(19):8444-55).The inventors previously demonstrated that GSK3β activation supports CAM-DR of leukemic blasts (De Toni F, et al.. Oncogene. 2006 May 25;25(22):3113-22). Accordingly, protection against oxidative stress of the leukemic inflammatory niche may be a key feature of LSCs.

However, no therapeutic tools for targeting leukemic stem cells is so far available for clinical use and thus there is still a need for an agent which would be specifically cytotoxic for adherent chemoresistant leukemic cells but not for normal hematopoietic cells.

Such innovative treatments would permit to efficiently prevent relapse and metastasis of cancer and more particularly cancer stem cell using a treatment which would be much better tolerated by patients than conventional chemotherapy or radiotherapy, which result in significant and sometimes severe adverse effects.

In particular, an innovative concept of therapy could be implemented, based on the inhibition of stem cell cancer which survives to drug-induced toxicity or radiotherapy, with or without conventional treatment of the bulk tumor.

### SUMMARY OF THE INVENTION:

The inventors have now unexpectedly found that compounds following formula (I) and in particular the rutin are specifically cytotoxic for adherent leukemic cells but not for normal hematopoietic cells. As shown in the following exemples, they advantageously target the GSK3β, Akt, FOX03a and MnSOD signaling pathways and induces apoptosis.

Consequently, in a first aspect, the present invention relates to a compound of formula (I) 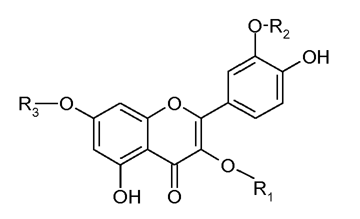 Wherein
R1, R2 and R3 represent, independently one from the others, a hydrogen atom, a saturated or unsaturated, linear or ramified C₁-C₅ alkyl group, an osidic residue or a derivative thereof,
with at least one of R1, R2 and R3 being obligatory an osidic residue or a derivative thereof,
and its salts, optical and geometric isomers or solvates,
for a use as selective cytotoxic agent for cancer stem cells.

As shown by the data submitted here-after, the compounds of formula (I) are advantageously selectively efficient against pathological adherent stem cells without altering normal stem cells ex vivo.

Within the meaning of the invention, the term "salt" refers to salt forms that are pharmacologically acceptable and substantially non-toxic. The salts of the invention include conventional acid-addition salts or base-addition salts formed from suitable non-toxic organic or inorganic acids or inorganic bases.

Within the meaning of the invention, the terms "optical and geometric isomers" intend to include enantiomers, stereoisomers, rotamers, tautomers and racemates of the compounds of formula (I) (where they exist). Isomers can be prepared using conventional techniques, either by reacting optically pure or optically enriched starting materials or by separating isomers of a compound of the formula (I).

Within the meaning of the invention, the term "solvate" refers to a molecular complex of a compound represented by formula (I) with one or more solvent molecules. Such solvent molecules are those commonly used in the pharmaceutical art, which are known to be innocuous to the recipient, e.g., water, ethanol, and the like.

In particular, the compounds of the invention are useful in the prophylaxis or treatment of diseases mediated by cancer stem cell.

Accordingly, the compounds of formula (I) may be used in the treatment of a cancer, in particular for the treatment of a solid tumour, a lymphoma or a leukemia, and more particularly of the acute myeloid leukemia.

They may be also used in preventing tumor relapse in a patient in need thereof.

They are also particularly useful for preventing solid tumor metastasis in a patient in need thereof.

In a second aspect, the invention relates to a pharmaceutical composition comprising an effective amount of at least a compound of formula (I) according to the invention in combination with at least one cancer agent different from a compound of formula (I), and in particular a secondary chemotherapeutic agent.

In a third aspect, the invention further relates to a method for treating a disease or condition comprising or arising from cancer stem cells, in particular solid tumor, lymphoma and leukemia and more particularly acute myeloid leukemia comprising the administration to a patient in need thereof of an effective amount of at least a compound of formula (I) as defined previously.

In another aspect, the invention relates to a method for alleviating or reducing the incidence of a disease state or conditions mediated by cancer stem cells comprising the administration to a patient in need thereof of an effective amount of at least a compound of formula (I) as defined previously.

The invention also relates to a method for preventing tumor relapse and/or solid tumor metastasis comprising the administration to a patient in need thereof of an effective amount of at least a compound of formula (I) as defined previously.

Moreover the present invention further concerns a method for specifically treating cancer stem cells and preserving normal stem cells ex vivo from a leukaemic or cancerous patient's tissue sample, comprising contacting said tissue sample with at least one compound of formula (I) according to the invention.

According to a particular embodiment, the compound of formula (I) is rutin or a derivative thereof.

As disclosed hereafter, such a compound may be extracted from Hammada scoparia.

Hammada scoparia is a medicinal plant already known for exhibiting anti-cancer, anti-inflammatory and anti-oxidant properties (Ben Salah H, et al., Chem Pharm Bull (Tokyo). 2002 Sep;50(9):1268-70).

Regarding rutin, which is a glycosylated flavonoid compound, it has only been disclosed in JP 9176011 for the treatment of cancer related with heat shock protein.

### DETAILED DESCRIPTION OF THE INVENTION:

### Definitions

As used herein
- "osidic residue" refers to monosaccharide, disaccharide or oligosaccharide and derivatives thereof like for example their deoxy derivatives and their products of oxidation like their related uronic compounds.
- "tumor" refers to an abnormal growth of tissue resulting from an abnormal multiplication of cells. A tumor may be benign, premalignant, or malignant {i.e., cancerous). A tumor may be a primary tumor, or a metastatic lesion.
- "cancer" and "cancerous" refer to or describe the physiological condition in mammals that is typically characterized by unregulated cell growth.
- "cancer stem cell(s)" refers to a cell that can be a progenitor of a highly proliferative cancer cell. A cancer stem cell has the ability to re-grow a tumor as demonstrated by its ability to form tumors in immunocompromised mice, and typically to form tumors upon subsequent serial transplantation in immunocompromised mice. Cancer stem cells are also typically slow-growing relative to the bulk of a tumor; that is, cancer stem cells are generally quiescent. In certain embodiments, but not all, the cancer stem cell may represent approximately 0.1 to 10% of a tumor.
- "stem cell cancer" refers to cancer which have been found to include cancer stem cells that drive the tumour growth and give rise to the variety of differentiated cells found in the malignancy. Examples of stem cell cancers of the invention include brain cancer, head and neck cancer, breast cancer, prostate cancer, colon cancer, ovarian cancer, skin cancer, hepatocellular cancer, pancreas cancer, lung cancer and leukemia. In a particular embodiment, the stem cell cancer is a leukemia. In another particular embodiment, the leukemia is acute myeloid leukemia.
- "tumor metastasis" means the process by which the cancer spreads from the place at which it first arose as a primary tumor to distant locations in the body. The cancer resulting from the spread of the primary tumor. Metastasis depends on the cancer cells acquiring two separate abilities - increased motility and invasiveness. Cells that metastasize are basically of the same kind as those in the original tumor. If a cancer arises in the lung and metastasizes to the liver, the cancer cells in the liver are lung cancer cells. However, the cells have acquired increased motility and the ability to invade another organ. The ability to invade another organ and to result with a metastasis showing morphological resemblance and similar gene expression profile to the primary tumor could reflect the unique capacities of cancer stem cells to initiate and to drive tumor growth.
- "tumor relapse" occurs after a variable latency from subclinical levels of residual disease, termed minimal residual disease, at the primary tumor site or the secondary metastatic sites. There cancer cells have been maintained in a dormant state until their reactivation. Tumor recurrence is often with increased aggressivity and is not always identical phenotypically to the original tumor. Tumor relapse frequently leads to the death of the patients. Tumor relapse is supposed to occur from the cancer stem cells which have capacities of resistance to current clinical treatments and to regrowth tumor.
- "patient" is intended for a human or non-human mammal affected or likely to be affected with a condition associated with a tumor. Said patient is preferably a human being.

### Compound of formula (I)

As stated previously, the compounds considered according to the invention are of the following formula: 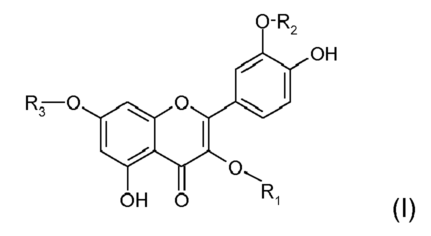 wherein
R1, R2 and R3 represent, independently one from the others, a hydrogen atom, a saturated or unsaturated, linear or ramified C₁-C₅ alkyl group, an osidic residue or derivatives thereof, with at least one of R1, R2 and R3 being obligatory an osidic residue or a derivative thereof.

According to one embodiment, at least two of R1, R2, and R3, and more particularly, all three of R1, R2, and R3 are obligatory an osidic residue or a derivative thereof.

The saturated alkyl group may be selected among the methyl, ethyl, n-propyl, isopropyl, n-butyl, isobutyl, ter-butyl and pentyl radicals.

The unsaturated alkyl group may be selected among the ethylenyl, n-propylenyl, isopropylenyl, n-butylene, isobutylenyl, ter-butylenyl and pentylenyl radicals.

More particularly, the alkyl group is in C₁-C₃, and in particular is a methyl radical.

According to the invention, "osidic residue" means radical issued from carbohydrates formed from one or several monosaccharide components joined together.

According to one embodiment, an osidic residue may be a monosaccharide, a disaccharide or an oligosaccharide radicals and derivatives thereof.

As used herein "monosaccharide" refers to simple sugars, carbohydrates which cannot be decomposed by hydrolysis. Examples of monosaccharide residue are heptoses, hexoses, and pentoses.

The term "hexose" refers to fructose, fucose, galactose, glucose, mannose, thamnose and sorbose.

The term "pentose" refers to arabinose, ribose, xylose and xylulose.

As used herein, the term disaccharide encompasses reducing disaccharides in which the monosaccharide components are bonded by hydroxyl groups and non reducing disaccharides in which the components bond through their anomeric centers.

Such disaccharide radical may be selected in the group consisting of sucrosyl, lactosyl, maltosyl, trehalosyl, cellobiosyl, gentobiosyl, isomaltosyl, kojobiosyl, laminaribiosyl, melibiosyl, nigerosyl, rutinosyl and xylobiosyl radical.

According to one embodiment, an osidic residue may be in particular a rotinosyl radical.

As stated previously, the term osidic residue also encompasses derivatives of the previous saccharides like their deoxy related compounds and their oxidized related compounds, like for example their uronic derivatives.

In a particular embodiment, R1 or R3 and more preferably R1 is an osidic residue.

According to one embodiment, at least one of R1, R2, and R3, and in particular R2 and/or R3, is/are a radical different from an osidic residue.

In another particular embodiment, the radical(s) different from the osidic residue is/are a hydrogen atom or a C₁-C₃ alkyl, and in particular is/are a methyl group.

In particular, the radical(s) different from the osidic residue is/are a hydrogen atom.

According to another specific embodiment, the osidic residue is a monosaccharide or disaccharide.

More preferably, the monosaccharide is a residue derived from glucose or rhamnose.

In another particular embodiment, the disaccharide is a residue issued from rutinose.

Thus, according to a preferred embodiment, R1 means a radical rutinosyl in formula (I) with preferably R2 and R3 being an hydrogen atom. Its formula may be as follows:

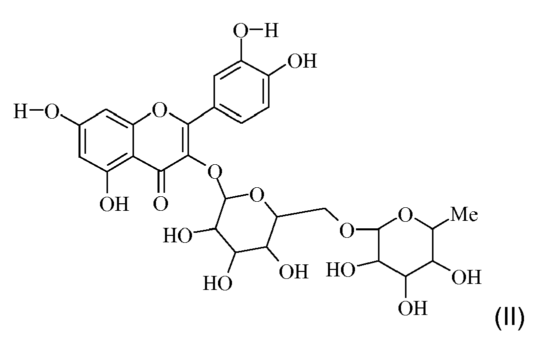

The compounds of the invention may be commercially available.

Alternatively, they may be extracted from plants species according to usual techniques of extractions known from the skilled artisan.

As stated previously, such a compound of formula (II), also called rutin, has been characterized by the inventors as a H. scoporia flavonoid.

More particularly, the inventors demonstrated that H. scoparia extract, and rutin, a H. scoporia flavonoid, have the capacities to specifically induce the cytotoxicity of adherent leukemic cells. Furthermore, as shown in the following examples, mostly, leukemic or normal cells in suspension were not significantly killed by the H. scoparia extracts.

Regarding rutin, it displayed also a cytotoxic effect specifically on adherent leukemic cells. Interestingly, among three AML patients whose cells have been experimented with rutin, two were responsive with a specific decrease of cell survival upon adhesion. By contrast with the injury of several leukemic cell lines and AML cells from patients, adherent normal hematopoietic cells from bone marrow or blood were spared.

Furthermore, the immature pool (CD34+) of leukemic cells from AML patient, supporting chemoresistance and risk of relapses, was particularly injured. The cytotoxicity of H. scoparia flavonoids was at least supported by apoptotic processes. Furthermore, the H. scoparia fractions #681 flavonoids-enriched, and rutin, inhibited GSK3β and Foxo3A, two anti-apoptotic signalling pathways.

Thus, as well as for the Parthenolide treatment proposed to eradicate LSCs (Guzman ML, et al.. Blood. 2005 Jun 1;105(11):4163-9), the cytotoxicity of H. scoparia flavonoids may involve pro-apoptotic mechanisms through the inhibition of NF-κB (here GSK3β-dependent, 2) and through the regulation of some specific reactive oxygen species.

### Therapeutic application:

Accordingly, the inventors have demonstrated that the compound of formula I may be specifically cytotoxic for adherent leukemic cells.

Since it appears that stem cell cancers of other cancers than acute myeloid leukemia also share the feature of adhesion, the present invention may be used for cancers stem cell in general. In effect, adhesive interactions between cancer stem cells and their environment are required to maintain stem cell capacities in different type of stem cell cancer (Jin L. Et al., Nat Med 2006, 12:1167; Hambardzumyan D. Et al., Genes Dev. 2008, 22:436; and Calabrese et al., Cancer Cell 2007, 11:69).

Indeed, there is increasing evidence that in other cancers, like in AML, the tumor clone is also maintained by the extensive proliferation and self-renewal of rare cancer stem cells.

According to an embodiment, a compound of the invention may be useful in the prophylaxis or the treatment of diseases mediated by cancer stem cells, more particularly in the prophylaxis or the treatment of a solid tumor, a lymphoma or a leukemia.

Said medicament/method avoids the generation of cancer cells issued from cancer stem cells, in particular, leukaemic-cells.

Accordingly, the compounds according to the instant invention, and in particular the compound of formula (II) are particularly useful for the treatment of leukaemia, breast cancers, brain cancer, prostate cancer, colon cancer, head and neck cancers, skin cancer, ovarian cancer, hepatocellular cancer, pancreas cancer, and lung cancer.

As stated previously, the invention further relates to the use of a compound of formula (I) according to the invention for the treatment of a stem cell cancer in combination with another anti-cancer agent or another anti-cancer treatment.

As mentioned above, a differentiation therapy needs the use of chemotherapy in order to eradicate cancer stem cells. The use of a compound of formula (I) according to the invention induces the alteration of leukaemic stem cells without the requirement of any chemotherapy.

According to the present invention, such compound of formula I would be particularly useful in combination with standard therapy, in any clinical situation in which a strong response is expected from standard therapy, and in which, nevertheless, relapses are frequent.

By "another anti-cancer treatment" is meant any other suitable treatment approved for cancer treatment. In particular, said other anti-cancer treatment may be selected from the group consisting of chemotherapy, surgery, radiotherapy, hormonotherapy, and/or immunotherapy.

In the case of chemotherapy, at least one secondary chemotherapeutic agent may be used. Such an agent may be selected from the group consisting of growth inhibitory agents (defined as compounds or compositions which inhibit growth of a cell, either *in vitro* or *in vivo*) and cytotoxic agents (defined as compounds or compositions which inhibits or prevents the function of cells and/or causes destruction of cells).

In a particular embodiment of the invention, a secondary chemotherapeutic agent may be selected from the group consisting of taxanes, topoisomerase II inhibitors, DNA alkylating agents, anti-metabolite, anti-tubuline, vinca alkaloids, intercalating agents and platinium salts.

In a more particular embodiment of the invention, a secondary chemotherapeutic agent may be selected from the group consisting of: paclitaxel, docetaxel, doxorubicin, epirubicin, daunorubicin, etoposide, bleomycin, tamoxifen, prednisone, dacarbazine, mechlorethamine, methotrexate, 5-fluorouracil, anthracyclines, adriamicin, vinblastine, vincristine, vinorelbine, topotecan, carboplatin, cisplatin, permetrexed, irinotecan, gemcitabine, gefinitib, erlotinib, fludarabin, ifosfamide, procarbazine, mitoxanthrone, melphalan, mitomycin C, chlorambucil, cyclophosphamide and platinium salts.

Of course, any suitable combination of chemotherapeutic drugs may be used, depending on the type of cancer.

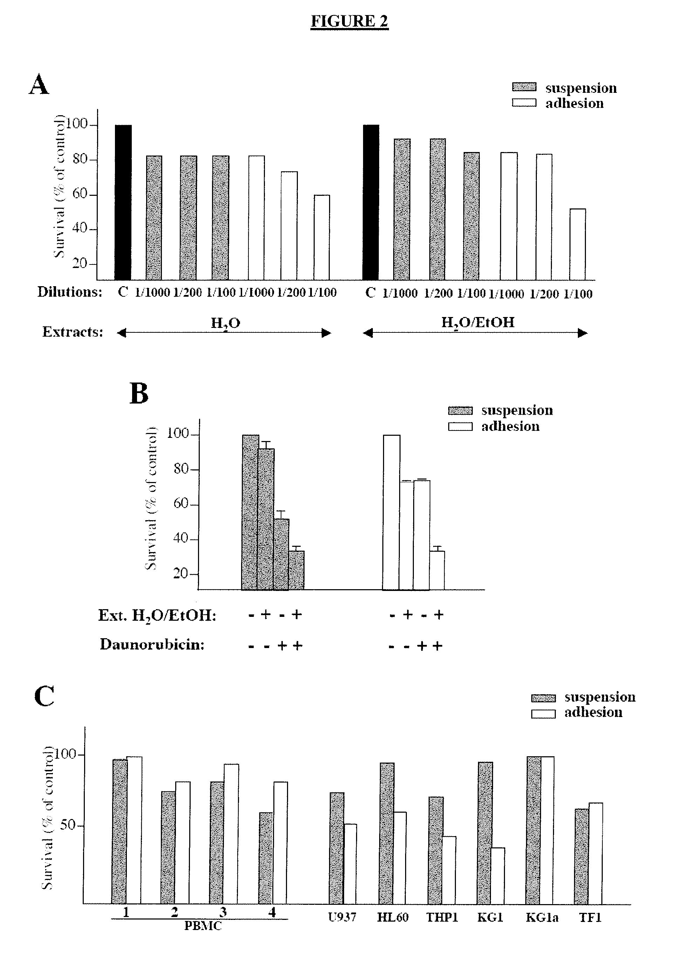

The figure 2B illustrates this combination between a standard therapy with an antiproliferative agent (daunorubicin) and the cytotoxic action of H. scoparia extract for adherent leukemic cells which show their synergic action. The toxicity of daunorubicin was greatly increased by H. scoparia extracts and CAM-DR was abolished.

A compound of formula (I) according to the invention may be administered at doses from approximately 200 mg to 1 g by day (adult 70 kg). The number of cures may be increased or reduced and/or repeated (over time) to improve the efficacy of the medicament.

Since the compound of formula (I) is not toxic, its dosage may be easily increased and adapted to the patient.

The production of the medicament may be in any suitable pharmaceutical formulation, and particularly in the form of tablets, granules, capsules, powder forms, suspension, oral solutions and solutions for injection. Administration may be preferably performed *per os* (oral administration).

The medicament, used according to the invention, may also further comprise any suitable compound or excipient adapted to the desired formulation, particularly any pharmaceutically inert vehicle.

The present invention is illustrated by the following examples, given purely for illustrative purposes, with reference to the following figures.

### FIGURE LEGENDS:

Figure 1 : Flavonoid precipitation protocol from Hammada scoparia leaves.
Figure 2 : Effect of aqueous extracts from Hammada scoparia towards leukemic cells (U937, HL60, THP1, KG1, KG1a, TF1).
Figure 3 : Effect of the flavonoid Rutin on adherent leukemic cells.

### MATERIAL AND METHODS

### A- Cells

Leukemic cell lines U937, HL-60, TF1, THP1 and KG1, KG1a were purchased from the German Collection of Microorganisms and Cell Cultures (Braunschweig, Germany) and grown in RPMI-1640 containing 10% or 20% (TF1) FCS and lscove's modified Dulbecco's medium (IMDM) containing 20% FCS, respectively, in the presence of 100 units/mL penicillin/100 µg/mL streptomycin at 37°C and 5% CO₂. Fresh AML cells were obtained from bone marrow of patients upon informed consent, using Ficoll-Hypaque density-gradient centrifugation. Bone marrow samples contained more than 80% leukemic blasts after processing. For *in vitro* experiments, blasts were resuspended in IMDM. All patients were diagnosed according to WHO classification at the Hematology Department of Toulouse University Hospital (France). Normal bone marrow CD34+ and CD34- hematopoietic cells were harvested from healthy volunteers upon informed consent and obtained after positive selection of CD34-expressing cells by the means of immunomagnetic separation column (Miltenyi Biotech, Bergisch Gladbach, Germany). Fresh blood samples were collected from healthy adult donors and PBMC (Peripheral Blood Mononuclear Cells) were prepared on a Ficoll-Paque density gradient by centrifugation (800 g, 30 min at room temperature). Collected CD34+/CD34- bone marrow cells and PBMC were finally diluted in IMDM and RPMI 1640 media, respectively, supplemented with penicillin and streptomycin.

### B- Scoparia extracts & chromatographic procedures

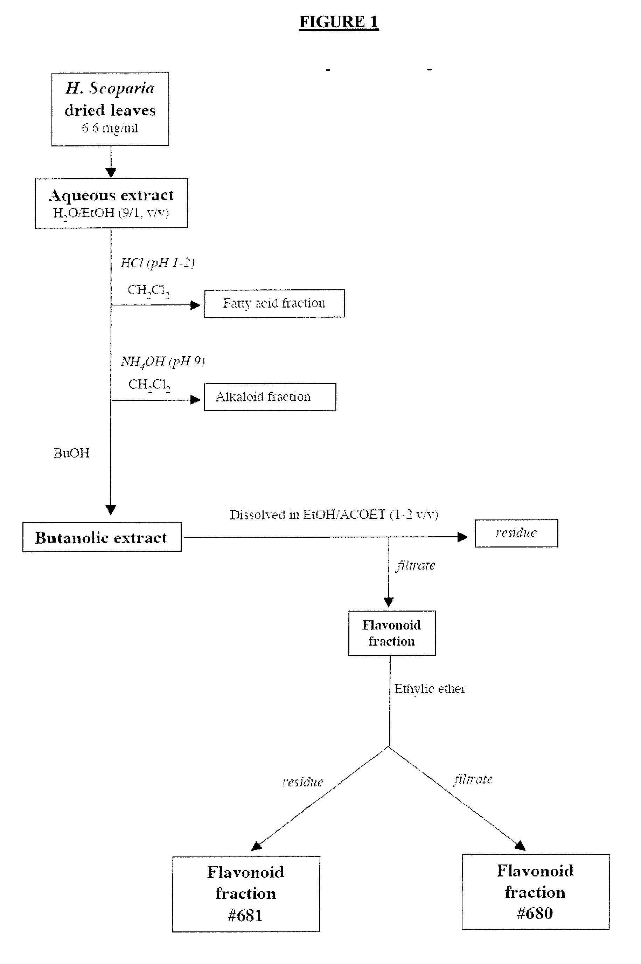

Leaves of H. scoparia (Chenopodiacea) were collected in southern Tunisia in June 2006. Leaves of H. scoparia were extracted by incubation with H₂O or EtOH/H₂O (1-9 v/v) under stirring for 24 h and then extracts were dried by lyophilisation. EtOH/H₂O extract was mixed with CH₂Cl₂, and the residue was treated with BuOH. The dried butanolic extract was used for flavonoids precipitation assay (Fig. 1). Each fraction was then evaporated in a vacuum rotary evaporator, weighted and kept in dark at 4°C until use.

### C- Adhesion and cytotoxic assays

Adhesion assays were performed in 96-well plates coated overnight at 4°C with 40 µg/mL of human fibronectin (Roche Molecular Biochemicals, Mannheim, Germany) in a final volume of 50 µL in PBS. Each well was blocked with 1% fatty acid-free BSA in PBS, 1 h at room temperature and washed with PBS before seeding the cells. Cells were diluted at 3x10⁵/mL overnight and then were serum starved for 1 h at 1x10⁶/mL, incubated or not with plant extract/fraction and purified alkaloid or flavonoid compounds. Control assays were treated with corresponding solvents (water, water/ethanol, DMSO) at a 1/100 dilution. Cells were then allowed to adhere on fibronectin (10⁵ cells/well) for 1 h at 37°C or maintained in suspension. In some experiments, both cells in suspension and adherent were treated with 0.5 µM daunorubicin (DNR) (Laboratoire Roger Bellon, Neuilly-sur-Seine, France) for 1 h at 37°C. At the end of the incubation period, cells were washed and incubated in serum containing medium for 24 h at 37°C. Cell viability was then quantified by methyl thiazolyl tetrazolium (MTT) assay (Sigma) or by the Annexin V-FITC detection kit from BD PharMingen. Experiments were performed in triplicate and results were evaluated as the percentage of surviving cells compared to the control group.

### EXAMPLE 1

### Cytotoxicity of aqueous extracts from H. scoparia towards adherent leukemic cells

Aqueous extracts were prepared from dried H. scoparia leaves and serial dilutions were assayed for cytotoxicity over leukemic cells either in suspension or in adhesion following CAM-DR protocol.

More specifically, aqueous extracts from Hammada scoparia are cytotoxic for leukemic cells.

As shown in Fig.2A, pretreatment of leukemic cells U937 with aqueous extracts from H. scoparia (water or ethanol 1/water 9 v/v) induced a dose-dependent cytotoxicity of adherent U937 (40-50% decrease of survival). The survival of leukemic cells in suspension was not significantly affected.

The cytotoxicity induced by daunorubicin treatment was strongly increased by the H. scoparia aqueous extract both in suspension and in adherent conditions (Fig. 2B). The cell protective effect conferred by adhesion (CAM-DR) was abrogated by the pretreatment of cells with the H. scoparia extract. Different leukemic cell lines and normal hematopoietic cells were tested, and most leukemic cell lines displayed stronger sensitivity to the treatment with the H. scoparia aqueous extract in adherent conditions (Fig. 2C). Nevertheless, the highly chemoresistant cell line KG1a remained unchanged by the treatment with the H. scoparia extract as above. Normal hematopoietic cells from peripheral blood (PBMC) have not displayed a strong cytotoxic response to the plant extract treatment (Fig. 2C). It should be noted that, in all conditions, adhesion capacities of leukemic cells were unaffected by the treatment with H. scoparia extracts.

### EXAMPLE 2

### Effects of flavonoid-enriched fractions from Hammada scoparia on cell survival of U937 and of PBMC

Further separation of the H. scoparia aqueous extract by butanol (BuOH) extraction step provided the WEB extract as described for Fig.1. The extract obtained with butanol (but not with dichloromethane, not shown) displayed strong cytotoxic effects on adherent cells in our assays. Precipitation of flavonoids was then realized as described in the Material and Methods section allowing the obtention of two flavonoid-enriched fractions #680 and #681 (Fig.1).

Effects of flavonoid-enriched fractions from Hammada scoparia on cell survival of U937 and of PBMC

The results are submitted in the following tables I and II.

**Table I: U937**

| Flavonoid fraction | Dilution | Cell survival (%) | |
|---|---|---|---|
| | | Suspension | Adhesion |
| **#681** | 1/50 | 100 | 53 |
| | 1/100 | 100 | 55 |
| | 1/500 | 100 | 60 |
| | 1/1000 | 100 | 63 |
| | 1/2000 | 100 | 93 |
| | 1/4000 | 100 | 98 |

| | | | |
|---|---|---|---|
| (Initial concentration of #681 fraction: 10mg/mL.) | | | |

**Table II: PBMC**

| Flavonoid fraction | Dilution | Cell survival (%) | |
|---|---|---|---|
| | | Suspension | Adhesion |
| **#681** | 1/50 | 98 | 100 |
| | 1/100 | 94 | 95 |
| | 1/500 | 94 | 94 |
| | 1/1000 | 98 | 91 |
| | 1/2000 | 95 | 93 |
| | 1/4000 | 95 | 94 |

| | | | |
|---|---|---|---|
| (Initial concentration of #681 fraction: 10mg/mL.) | | | |

The #681 fraction induced cell death of adherent leukemic cells (Table I) whereas the #680 fraction was cytotoxic for both leukemic cells in adhesion or in suspension (not shown).

By contrast, PBMC were not killed by the #681 fraction treatment (Table II).

Furthermore and as stated in following table III, effects of the flavonoid-enriched #681 fraction from Hammada scoparia has also been listed on cell survival of normal hematopoietic and leukemic cells from patients.

In this further experiment, flavonoids were precipitated from aqueous extract of *H. Scoparia* as described in Fig. 1. The #681 flavonoid-enriched fraction was then incubated at different dilutions with leukemic or normal hematopoietic (CD34⁺) cells from patients before adhesion onto fibronectin. Cells survival was measured by Annexin V/PI labelling at 24h after serum addition. Data submitted in the following table III are representative of two independent experiments and expressed as % of respective control in adhesion or in suspension. Initial concentration of #681 fraction: 10mg/mL.

**Table III**

| Cells | Dilution #681 fraction | Cell survival (%) | |
|---|---|---|---|
| | | Suspension | Adhesion |
| **CD34⁺** | 1/100 | 95 | 90 |
| | 1/500 | 95 | 88 |
| | | | |
| **AML Patient** | 1/100 | 100 | 60 |
| | 1/500 | 100 | 78 |
| | 1/1000 | 100 | 82 |

Interestingly, leukemic cells from AML patients were efficiently killed in adhesion whereas normal immature hematopoietic cells (CD34+) were not significantly sensitive to the #681 treatment (Table III).

### EXAMPLE 3

### Characterization of the flavonoids present in the H. Scoporia and of their respective effect on adherent leukemic cells

Analysis of the #681 extract by TLC suggested that its composition was a mixture of flavonoids and of few alkaloids (not shown). Close examination of the MS and MSn spectra obtained from the LC-UV profile confirmed the presence of two groups of naturally occurring derivatives: alkaloids and flavonol glycosides. Comparison with the published chemistry of H. scoparia (Ben Salah H, et al. Chem Pharm Bull (Tokyo) 2002 Sep;50(9):1268-70; Benkrief R, et al. Annales Pharmaceutiques Françaises 1990;48(4):219-224; EI-Shazly A, et al. Zetzchrift Fur Naturforschung C-a Journal of Biosciences 2003;58(7-8):477-480; EI-Shazly AM, et al. Pharmazie 2005;60(12):949-952) led to the identification 14 compounds (9 alkaloids and 5 flavonol glycosides). Thus, the alkaloid part of the chromatogram revealed the presence of six tetrahydroisoquinoline derivatives (salsolinol, isosalsoline, N-methylisosalsoline, salsolidine, carnegine and N-methylcorydaldine) described in H. scoparia by Benkrief et al. (Benkrief R, et al. Annales Pharmaceutiques Françaises 1990;48(4):219-224) and EI-Shazly et al. in 2003 (EI-Shazly A, et al. Zetzchrift Fur Naturforschung C-a Journal of Biosciences 2003;58(7-8):477-480), tryptamine and N-methyltryptamine also described in (Benkrief R, et al. Annales Pharmaceutiques Françaises 1990;48(4):219-224) and a newly detected alkaloid in Hammada, norsynephrine. In the flavonoid part of the chromatogram, three isorhamnetin glycosides and two quercetol glycosides were detected. The presence of isorhamnetin 3-O-β-D-xylopyranosyl-(1"'->3"')-α-L-rhamnopyranosyl-( 1"'->6"')-β-D-galactopyranoside and Isorhamnetin-3-O-β-D-robinobioside, ever been described in (Ben Salah H, et al. Chem Pharm Bull (Tokyo) 2002 Sep;50(9):1268-70) and (Benkrief R, et al. Annales Pharmaceutiques Françaises 1990;48(4):219-224) respectively, has been confirmed. In contrast, close examination of MS-MS spectra structures led to the identification of quercetin-xylose-rhamnose-galactose, quercetin-rhamnose-glucose commonly called rutin and isorhamnetin-xylose-galactose.

The anti-leukemic activity of N-methylisosalsoline, tryptamine, norsynephrine and rhamnetin has been checked in vitro but none of these compounds displayed a specific proapoptotic effect on adherent U937 cells (not shown).

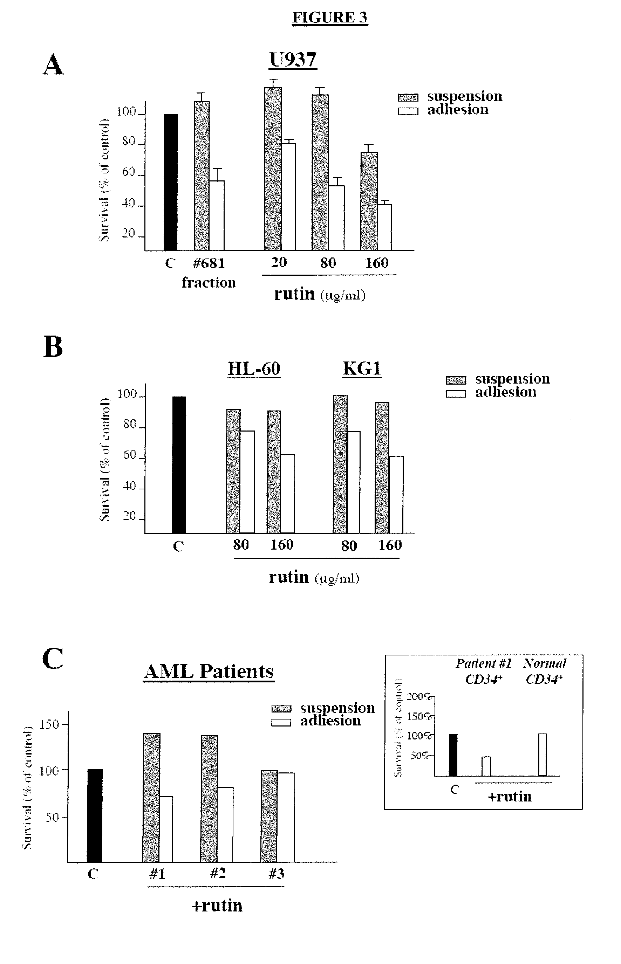

By contrast, the flavonol glycoside rutin showed qualitatively a similar cytotoxicity to the #681 fraction on leukemic adherent cells (Fig. 3A). Of note, addition of rutin or of #681 fraction to U937 cells already adherent onto fibronectin is still able to trigger cell death (-30%, not shown). Also, rutin killed efficiently HL-60 and KG1 cell lines (Fig. 3B). Importantly, adherent cells from AML patients were mostly injured by rutin treatment and adherent immature leukemic cells (CD34+) were strikingly committed to apoptosis by contrast with normal CD34+ cells (Fig. 3C).

Thus, these results suggest that the specific cytotoxic activity of H. scoparia extracts against adherent leukemic cells could be partially supported by rutin.

## Claims

1. A compound of formula (I) 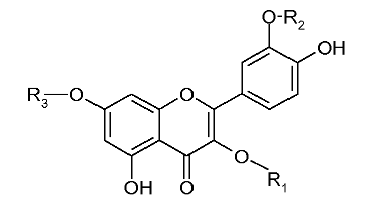 wherein
R1, R2 and R3 represent, independently one from the others, a hydrogen atom, a saturated or unsaturated, linear or ramified C₁-C₅ alkyl group, an osidic residue or a derivative thereof,
with at least one of R1, R2 and R3 being obligatory an osidic residue or a derivative thereof,
and its salts, optical and geometric isomers or solvates,
for a use as selective cytotoxic agent for cancer stem cells.

2. The compound of claim 1, wherein R1 is an osidic residue chosen in the group consisting of the monosaccharide, disaccharide, oligosaccharide radicals and derivatives thereof.

3. The compound according to anyone of claims 1 to 2, wherein R1 is a disaccharide radical in particular selected in the group consisting of sucrosyl, lactosyl, maltosyl, trehalosyl, cellobiosyl, gentobiosyl, isomaltosyl, kojobiosyl, laminaribiosyl, melibiosyl, nigerosyl, rutinosyl and xylobiosyl radical.

4. The compound according to anyone of claims 1 to 3, wherein R1 is a rutinosyl radical.

5. The compound according to anyone of claims 1 to 4, wherein the radical(s) differs from the osidic residue.

6. The compound according to anyone of claims 1 to 5, wherein the radical(s) different from the osidic residue is/are a hydrogen atom.

7. The compound according to anyone of claims 1 to 6 being of formula II:

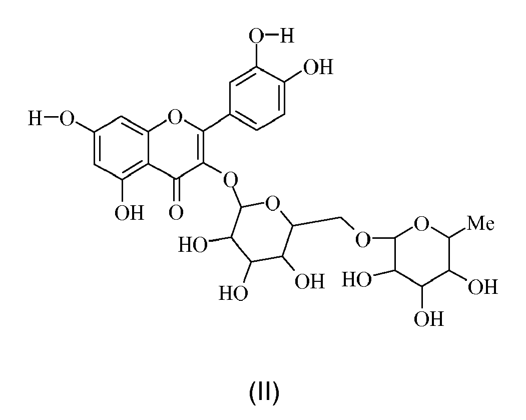

8. The compound according to anyone of claims 1 to 7 for use in the prophylaxis or treatment of diseases mediated by cancer stem cells.

9. The compound according to anyone of claims 1 to 8 for use in the treatment of a cancer.

10. The compound according to the previous claim wherein the cancer is a solid tumor, a lymphoma or a leukemia.

11. The compound of claim 10, wherein said cancer is selected from the group consisting of leukemia, breast cancers, brain cancer, prostate cancer, colon cancer, head and neck cancers, skin cancer, ovarian cancer, hepatocellular cancer, pancreas cancer, and lung cancer.

12. The compound of anyone of the claims 1 to 11 for the treatment of acute myeloid leukaemia.

13. The compound according to anyone of claims 1 to 12 for use in preventing tumor relapse in a patient.

14. The compound according to anyone of claims 1 to 13 for use in preventing solid tumor metastasis in a patient.

15. A pharmaceutical composition comprising an effective amount of a compound according to anyone of claims 1 to 7 in combination with at least one cancer agent different from a compound of formula (I) and in particular a secondary chemotherapeutic agent.

16. The pharmaceutical composition according to the previous claim wherein the secondary chemotherapeutic agent is selected in the group consisting of: paclitaxel, docetaxel, doxorubicin, epirubicin, daunorubicin, etoposide, bleomycin, tamoxifen, prednisone, dacarbazine, mechlorethamine, methotrexate, 5-fluorouracil, anthracyclines, adriamicin, vinblastine, vincristine, vinorelbine, topotecan, carboplatin, cisplatin, permetrexed, irinotecan, gemcitabine, gefinitib, erlotinib, fludarabin, ifosfamide, procarbazine, mitoxanthrone, melphalan, mitomycin C, chlorambucil, cyclophosphamide and platinium salts.
